# EUROPEAN PATENT APPLICATION

(11) **EP 4 331 574 A1**
(43) Date of publication of application: **06.03.2024**
(21) Application number: 23192357.4
(22) Date of filing: 21.08.2023
(51) Int. Cl.: A61K 9/28

(54) **TABLETS BASED ON SODIUM BUTYRRATE CONTAINING AN INNNOVATIVE GASTROPROTECTIVE COATING**

(30) Priority: 29.08.2022 IT 202200017745
(71) Applicant: Unifarco S.p.A., 32035 Santa Giustina (BL) (IT)
(72) Inventor: Gravier, Gabriele, Liscate MI (IT); Baratto, Giovanni, Santa Giustina BL (IT); Casanova, Elena, Santa Giustina BL (IT); Francescato, Stefano, Santa Giustina BL (IT)
(74) Representative: Perani & Partners S.p.A.

(57) **Abstract**

Coated tablets containing as active ingredient butyric acid, a pharmaceutically acceptable salt thereof, or a mixture of both,
comprising a core in which said active ingredient is dispersed and a gastroprotective coating wherein said gastroprotective coating comprises or consists of:
A) An intermediate coating or pre-coating that wraps the core and is in contact with the latter and said coating A) comprises or consists of:
a) A film-forming polymer selected from hydroxypropyl cellulose and polyvinyl alcohol;
b) A plasticizer chosen from polyethylene glycol, glycerol;
c) An opacifying agent selected from calcium carbonate, titanium dioxide;
d) An antiadhesive agent selected from talc, magnesium stearate, calcium stearate, stearic acid, microcrystalline cellulose;

B) An outer coating wrapping said intermediate coating or pre-coating A) comprising or consisting of the following components:
e) A film-forming polymer selected from ethyl cellulose or shellac;
f) A plasticizer selected from: triglycerides of medium chain fatty acids, triethyl citrate
g) A thickening agent selected from: sodium alginate, corn starch;
h) A lubricating agent selected from oleic acid, stearic acid or salts thereof.

## Description

### TECHNICAL FIELD

The present invention relates to tablets containing as active ingredient butyrate provided with an innovative gastroprotective coating.

### BACKGROUND OF THE INVENTION

Short chain fatty acids are known to have an aliphatic chain of one to five carbon atoms and are identified with the acronym SCFA.

Only a minimal percentage of SCFA is introduced with the diet, while most is the result of the anaerobic fermentation of the fibres present in ingested foods, in particular pectin and undigested starch, by the bacteria that colonise the colon. The fermentation leads to the formation of acetate, butyrate, propionate, hydrogen, carbon dioxide and other SCFAs in modest amounts.

In particular, butyric acid together with propionic acid represent about 90-95% by weight of the short chain fatty acids present within the large intestine. Furthermore, among the short chain fatty acids, butyrate represents, together with glutamine, the main energy source of the colon cells. The deficiency of butyrate in the large intestine consequently represents a major cause of atrophy of the intestinal mucosa and inflammatory states of the intestinal mucosa. Furthermore, SCFA deficiency, in particular butyric acid, has been shown to increase the risk of developing intestinal tumours, at the level of the colon.

Conversely, it has been documented that the increase in butyric acid values in the intestinal area plays a preventive and treatment action on some intestinal diseases such as colorectal cancer, both infectious and non-infectious colitis, ulcerative colitis, ulcerative rectocolitis, Crohn's disease and irritable bowel syndrome (IBS).

Furthermore, it has been found that the presence of physiological or higher levels of butyrate in the colon has an influence on the body's immune response and is capable of modulating the immune response.

Furthermore, some scientific studies document the role of butyric acid in inhibiting fluid loss in mammals affected by intestinal infections, particularly those affected by cholera.

Because of its specific anti-inflammatory and mucosal repair action, butyric acid is also used in the treatment of irritable bowel syndrome (IBS) or Inflammatory Colitis, a disease characterized by the presence of inflammation of the mucosa of the colon that is accompanied by manifestations of diarrhoea and bloating.

In cases of a reduced or insufficient endoluminal concentration of butyric acid, diet supplementation with an exogenous amount of butyric acid is therefore recommended.

The supplementation of butyric acid in the diet thus finds application in the treatment of the diseases mentioned above.

Exogenous butyric acid is generally administered in the form of soluble alkali metal salts. However, it has been found that the administration of butyric acid and the salts thereof suffers from two types of problems. The first is represented by the penetrating smell of this molecule, which makes it particularly unpleasant. This inconvenience may cause the patient to discontinue treatment or its supplementation in the dietary regimen.

A second drawback is that butyric acid is absorbed mainly in the initial or middle portion of the gastrointestinal tract.

Traditional tablets containing butyric acid, coming into contact with gastric juices, tend to dissolve in the stomach, therefore the active ingredient does not reach the part where its action is required. Consequently, the amount of butyric acid that reaches the intestine is inadequate to carry out an effective therapeutic or preventive activity of the aforementioned diseases.

Therefore, there is currently a need for formulations of butyric acid with controlled release along the intestinal tract.

In Italian patent application 102018000005908, a tablet with gastro-resistant coating based on anionic polyacrylates is described, and in which the core, consisting mostly of the active ingredient and a mixture of cellulose and hydroxypropylmethylcellulose, is prepared for dry mixing and subsequent compression.

The application of this type of gastro-resistant coating has several drawbacks.

Firstly, the fact that it is applied by spraying an aqueous solution of said polyacrylates. As is known, sodium butyrate in the presence of even small amounts of water tends to hydrolyse, releasing butyric acid, which is solely responsible for the bad odour. Thus, it tends to disintegrate already during the application of the coating.

This implies a series of problems because the cores can have a much lower weight than that desired. Furthermore, the equipment, in which the coating is applied, must be subjected to several long cleaning cycles to eliminate any traces of butyric acid. Even very low concentrations of butyric acid can pollute the production of tablets of the same type.

These cleaning operations of the equipment inevitably lengthen the industrial production times of this type of tablet, to the detriment of the process economy.

To this must also be added the fact that these polymers of a purely synthetic nature are difficultly removed from the human body; therefore, they can accumulate in the liver and/or kidneys.

The need is therefore felt to provide a gastroprotective coating which does not present the problems of the tablets disclosed in the aforesaid patent application IT102018000005908.

### SUMMARY OF THE INVENTION

The Applicant has now found that it is possible to overcome the aforesaid drawbacks thanks to the tablet object of the invention containing as active ingredient butyric acid, a pharmaceutically acceptable salt thereof comprising or consisting of a core in which said active ingredient is dispersed and a gastroprotective coating in which said gastroprotective coating comprises or consists of:
A) An intermediate coating or pre-coating that wraps the core and is in contact with the core and comprises or consists of:
   a) A film-forming polymer selected from hydroxypropyl cellulose or polyvinyl alcohol;
   b) A plasticizer chosen from polyethylene glycol, glycerol;
   c) An opacifying agent selected from calcium carbonate, titanium dioxide;
   d) An antiadhesive agent selected from talc, magnesium stearate, calcium stearate, stearic acid, microcrystalline cellulose.
B) An outer coating wrapping said intermediate coating A) or pre-coating, said coating B) comprising or consisting of the following components:
   e) A film-forming polymer selected from ethyl cellulose or shellac;
   f) A plasticizer selected from: triglycerides of medium chain fatty acids, triethyl citrate
   g) A thickening agent selected from: sodium alginate, corn starch,
   h) A lubricating agent selected from oleic acid, stearic acid or salts thereof.

In fact, thanks to the process that we will disclose in more detail in the present disclosure, the formation of the intermediate coating A) is obtained thanks to the nebulization of HPC in ethanol, cancelling the disintegration of the core.

However, this result is also obtained due to the presence of the outer layer B). The applicant has in fact found that, thanks to both coatings, the sodium butyrate is practically not released at the gastric level, since the amounts of butyrate released at the gastric level are well below 5%.

In contrast, at intestinal pH (6.8) the tablet is completely dissolved in 8 hours (see dissolution tests below).

### DETAILED DESCRIPTION OF THE INVENTION

For the purposes of the present invention, the definitions "comprising" and "containing" envisage the possible presence of further components beyond those mentioned after such a definition.

For the purposes of the present invention, the definition "consisting of" excludes the presence of further components beyond those mentioned.

Preferably each coating A) and B) constitutes between 3.5 and 4.5% by weight, preferably 4% by weight of the total weight of the core in the tablets object of the present invention.

Preferably in the gastro-resistant tablets according to the present invention the film-forming polymer a) is hydroxypropylcellulose, more preferably it is present at a concentration between 50 and 70%, even more preferably it is 60% by weight of the total weight of said coating A), the plasticizer b) is a polyethylene glycol preferably with an average weight molecular weight of 6000 Da, more preferably it is present at concentrations between 8 and 12%, even more preferably it is 10% by weight of the total weight of said coating, the opacifying agent c) is preferably calcium carbonate, more preferably present at concentrations between 17 and 23%, even more preferably it is 20% by weight of the total weight of said coating A); the antiadhesive agent d) is preferably talc, more preferably it is present at concentrations between 17 and 23%, even more preferably it is 20% by weight of the total weight of the film A).

Preferably in the tablets object of the present invention, the film-forming polymer e) is ethylcellulose, more preferably it is present at concentrations between 54 and 74%, even more preferably it is 64% by weight on the total weight of said coating B); the plasticizer f) is preferably a mixture of medium-chain fatty acid triglycerides, more preferably at a concentration between 11.5 and 14.5%, even more preferably it is 13.5% by weight on the total weight of said coating B); the thickening agent g) is preferably sodium alginate and is more preferably present at concentrations between 13 and 17%, even more preferably it is 15% by weight on the total weight of said coating B); the lubricating agent h) is preferably stearic acid and is more preferably present at concentrations between 6.3 and 8.8%, even more preferably it is 7.5% by weight on the total weight of said coating B.

The medium chain fatty acid triglyceride mixture defined by the acronym MT (Medium triglycerides) contains medium chain fatty acids esterified with glycerol.

The medium chain carboxylic acids that resulted in esterification with glycerol are C6:0 caproic acid, C8:0 caprylic acid; C10:0 capric acid;
C12:0 lauric acid.

A further object of the present invention is the process of preparing coated tablets, further object of the present invention.

This process comprises or consists of the following steps:
I) at room temperature a suspension comprising components a), b), c) and d) in ethanol is prepared, said suspension having a total solids content comprised between 16 and 24%, preferably with a content comprised between 15 and 20% by weight on the total weight of said composition;
II) the coating A) is applied by nebulizing the solution obtained in the previous stage on the tablet core previously heated to a temperature comprised between 34 and 36°C, preferably to 35°C, maintaining said temperature until the suspension to be nebulized is exhausted
III) the coated product is cooled in stage II);
IV) at room temperature an aqueous suspension of components e), f) g) and h) is prepared by first dissolving in water sodium alginate g) and part of stearic acid h) for 30 minutes at room temperature then ethyl cellulose e) the triglycerides of medium chain fatty acids f) and the remaining amount of stearic acid h) for approx. 20 minutes, said aqueous suspension having a total concentration of dry e), f) g) and h) on the total weight of the composition comprised between 8 and 20%, preferably 12% by weight on the total weight of the aqueous suspension;
V) The coating B) is applied by nebulizing the aqueous suspension on the coated tablet coming from stage III) previously heated to a temperature comprised between 38 and 45°C, preferably to 42°C, keeping it at the aforementioned temperature until the aqueous suspension to be nebulized is exhausted.

As already anticipated, a further advantage of the present invention resides in the aforesaid process for the preparation of the intermediate coating A) in the fact that the nebulization of the components a) -d) occurs in ethanol, instead of in water. Thereby, the disintegration of the core and the release of considerable amounts of active ingredient also at the gastric level are considerably reduced.

In any case, only the coated tablets according to the present invention remain intact at pH 1 for 2 hours, while at pH greater than or equal to 6.8 they completely release the active ingredient in a time between 1 and 8 hours.

This type of innovative coating can be applied to any type of core in which butyric acid, a pharmaceutically acceptable salt thereof, or mixtures of both are dispersed.

However, it is preferably applied on a tablet, described and claimed in Italian patent application No. 102022000017748 in the name of Sila S.r.l. filed on the same day as the present patent application, the core of which has a resistance greater than 200 N.

The preparation examples of the coated tablets object of the present invention and the release tests of the formulation object of the present invention are merely reported by way of nonlimiting illustration.

### Example 1

Preparation of coating A) a mixture is prepared having the following composition:
a) HPC 60%
b) Peg 10%
c) Calcium Carbonate 20%
d) Talc 20%

Ethanol is added to have a suspension with a total solids amount understood as component a) + c) + d) (Peg b) is liquid) 2, of 17%.

The suspension containing sodium butyrate, prepared as described and claimed in the patent application in the name of Sila S.r.l. filed on the same day as the present patent application, the core of which has a resistance greater than 200N, and which has previously been preheated to the same temperature, is applied by nebulization at 35°C until the nebulized suspension is completely exhausted.

The coated tablet is then cooled and an aqueous suspension is prepared the dry components of which are as follows:
e) Ethylcellulose 64%
f) Medium chain carboxylic acid triglyceride mixture 13.5%
g) Sodium alginate 15%;
h) Stearic acid 15%;

An aqueous suspension is obtained with a total concentration of the aforesaid components in water equal to 12% by weight on the total weight of the aqueous suspension.

The aqueous suspension is nebulized at 42°C onto the core preheated to the same temperature, said core being as described and claimed in Italian patent application 102022000017748 in the name of Sila S.r.l. filed on the same day as the present patent application, the core of which has a resistance greater than 200 N.

A continuous film is obtained without abrasions, with active release parameters that respect the pre-set target (no release for 2 hours in pH 1, release at pH 6.8 as per Eu.Ph. section 2.9.1).

Example 1A. Example 1 is repeated with the only difference that a 12% aqueous suspension is used for the preparation of coating A).

As for the coating B), the same methods as in Example 1 and the same ingredients in the same concentrations are used.

The application of the coating A) by nebulization of an aqueous-based suspension gives substantial problems of dissolution of the active ingredient present in the cores during the coating.

In fact, there is a strong abrasion between the tablets, which is entirely counter-productive to the gastro-resistant coating, not ensuring a continuity of the fundamental film to isolate the core from the acidic pH of the gastric tract.

### GASTRO-RESISTANCE TEST AND DISSOLUTION TEST

The gastro-resistance and dissolution test procedure is summarized: it was performed on the coated tablet as described in example 1.
- Loading of 750 ml of 2M HCl into the basket.
- Addition of 3 tablets - sequential samples at 0, 0.5, 1 and 2 hours (37°C).
- 2M HCl is emptied and replaced with 750 ml phosphate buffer (50 mM Na2HPO4 brought to pH 6.8), keeping the tablets in the basket - sequential samples in the following 3, 4, 6 and 8 hours (37 °C).

Sampling fluids were centrifuged, diluted 1:20 in 0.1 M HCl and placed in vials for analysis. The analyses were performed in GC-MS.

Butyric acid (Sigma-Aldrich) was used as standard and for confirmation as reference, the same tablets were also used, chopped, extracted in water (acidic water and buffer) and diluted in 0.1M HCl to more likely reproduce the conditions maintained during the release and the matrix effect.

### Dissolution in 2M HCl acidic environment (pH < 2)

The data related to the gastro-resistance test are reported

| **Time** | **Tablet Example 1** |
|---|---|
| T0 | 0 |
| 30 min | 3.2 |
| 1 hour | 3.3 |
| 2 hours | 4.2 |

In the tested tablets, the release does not reach 5% after the first two hours at pH =1.

### Dissolution in phosphate buffer (pH 6.8)

The data related to the dissolution test carried out following the gastro-resistance test in the following hours are reported.

| **Time** | **Tablet Example 1** |
|---|---|
| 3 hours | 38.7 |
| 4 hours | 55.7 |
| 6 hours | 85.4 |
| 8 hours | 101.1 |

In phosphate buffer at pH 6.8, a high release increase is observed in the first two hours. At the end of 6-8 hours, the release is almost complete.

### Example 2 Industrialization

The product has been scaled up for about 250 kg of 1 g cores and prepared on a solid coating pan of 350 L Nicomac, such cores being as described and claimed in the patent application in the name of Sila S.r.l. filed on the same day of the present patent application, the core of which has a resistance greater than 200 N.

The following excipients were weighed for the pre-coating:
- Hpc: 6535 g
- Talc: 2178 g
- Calcium Carbonate: 2178 g
- Peg: 1416 g
- Ethanol: 58 L (46748 g)

The whole was mixed in a 70 L dissolver for about 40 minutes, during the process the suspension was allowed to stir slightly, at about 30% of the power of the dissolver.

After the pre-operating checks on the condition of the coating pan, 250 kg of the aforementioned cores were loaded.

The process is started with a heating step, bringing the batch to a temperature of about 35 degrees, by means of an air flow at about 60 degrees inside the machine.

Once the 35-degree checkpoint of the T of the cores was reached, the spraying step of the alcohol-based filming suspension began (HPC, talc, calcium carbonate, peg), maintaining a constant product temperature around 35°C (ranging from 34 to 36°C). The entire pre-coating suspension was sprayed in about 4 hours.

Once the pre-coating was finished, the batch was cooled, maintaining the rotation of the basket at 1 rpm.

Meanwhile, the second suspension for the top-coating was prepared as follows: dry concentrations of the following components:
e) ethylcellulose: 24,746.24 g
f) mixture of triglycerides with medium chain carboxylic esters 6,569.91 g
g) sodium alginate:7299.9 g
h) stearic acid: 3,649.95 g
71 litres (71000 g of water) are added to the aforesaid mixture.

The whole was mixed in a 120 L dissolver, first dissolving the sodium alginate g) and a part of stearic acid h) in water for about 30 minutes, then adding ethylcellulose e) and the mixture of medium chain fatty acid triglycerides f) and allowing the whole to mix for about another 20 minutes. first dissolving sodium alginate g) and part of stearic acid h) in water for 30 minutes at room temperature, then adding ethylcellulose e) medium chain fatty acid triglycerides f) and the remaining quantity of stearic acid h) for about 20 minutes.

The product in the coating pan is heated again, and brought to a temperature of about 42 degrees.

Once the 42-degree checkpoint of the product T was reached, the spraying step of the water-based filming suspension began, maintaining a constant product temperature around 42 degrees (with a range from 38 to 45). The entire top-coating suspension was sprayed in about 7 hours.

After the last processing, the batch was cooled and brought to a temperature of 30 degrees while maintaining the rotation of the basket at 1 rpm.

A sample was taken for internal analysis to confirm the data obtained in R&D, i.e., gastro-resistance as per Eu.Ph. section 2.9.1.

The results obtained in R&D were therefore also confirmed in scale-up up to a coating pan load of 250 kg.

## Claims

1. Coated tablets containing as active ingredient butyric acid, a pharmaceutically acceptable salt thereof, or a mixture of both,
comprising a core in which said active ingredient is dispersed and a gastroprotective coating wherein said gastroprotective coating comprises or consists of:
A) An intermediate coating or pre-coating that wraps the core and is in contact with the core and comprises or consists of:
a) A film-forming polymer selected from hydroxypropyl cellulose and polyvinyl alcohol;
b) A plasticizer chosen from polyethylene glycol, glycerol;
c) An opacifying agent selected from calcium carbonate, titanium dioxide;
d) An antiadhesive agent selected from talc, magnesium stearate, calcium stearate, stearic acid, microcrystalline cellulose;
B) An outer coating wrapping said intermediate coating or precoating A) and comprising or consisting of the following components:
e) A film-forming polymer selected from ethyl cellulose or shellac;
f) A plasticizer selected from: a mixture of triglycerides of medium chain fatty acids, triethyl citrate;
g) A thickening agent selected from: sodium alginate, corn starch;
h) A lubricating agent selected from oleic acid, stearic acid or salts thereof.

2. Gastro-resistant tablets according to claim 1, wherein the coating A) constitutes between 3.5 and 4.5% by weight, preferably 4% by weight, of the total weight of the core.

3. Gastro-resistant tablets according to claim 1, wherein the film-forming polymer a) is hydroxypropyl cellulose, the plasticizer b) is a polyethylene glycol, preferably with an average weight molecular weight of 6000 Da, the opacifying agent c) is calcium carbonate; the antiadhesive agent d) is talc.

4. Gastro-resistant tablets according to claim 3, wherein the hydroxypropyl cellulose is present at concentrations ranging from 50 to 70% by weight, preferably is 60% by weight of the total weight of said coating; the polyethylene glycol is at concentrations ranging from 8 to 12% by weight, more preferably 10% by weight of the total weight of said coating A); the calcium carbonate c) is present at concentrations ranging from 17 to 23% by weight, more preferably is 20% by weight of the total weight of said coating A) and talc d) is present at concentrations preferably comprised between 17 and 23% by weight, more preferably is 20% by weight of the total weight of the film A).

5. Tablets according to any one of claims 1-4, wherein the coating B) constitutes between 3.5 and 4.5% by weight, preferably 4% by weight of the total weight of the core.

6. Tablets according to any one of claims 1-5 wherein the film-forming polymer e) is ethyl cellulose, preferably present at concentrations ranging from 54 to 74%, more preferably is 64% of the total weight of said coating B); the plasticizer f) is a mixture of triglycerides of medium chain fatty acids preferably present at concentration comprised between 11.5 and 14.5%, more preferably is 13.5% of the total weight of said coating; the thickening agent g) is sodium alginate preferably present at concentrations ranging from 13 to 17% by weight, even more preferably is 15% by weight of the total weight of said coating B) and the lubricating agent h) is stearic acid and is preferably present at concentrations ranging from 6.3 to 8.8% by weight, more preferably is 7.5% by weight of the total weight of said coating B).

7. Process of application of the coating on the tablet according to claim 6 comprising the following steps:
I) at room temperature a suspension comprising components a), b), c) and d) in ethanol is prepared, said suspension having a total solids content comprised between 15 and 24% by weight preferably 15 to 19% by weight, still more preferably with a content of 17% by weight based on the total weight of said composition;
II) the coating A) is applied by nebulizing the solution obtained in the previous stage on the tablet core previously heated to a temperature comprised between 34 and 36°C, preferably to 35°C, maintaining said temperature until the solution to be nebulized is exhausted;
III) the coated product coming from stage II is cooled);
IV) at room temperature an aqueous suspension of components e), f) g) and h) is prepared by first dissolving in water sodium alginate g) and part of stearic acid h) for 30 minutes at room temperature then ethyl cellulose e) the triglycerides of medium chain fatty acids f) and the remaining amount of stearic acid h) is added for approx. 20 minutes, said aqueous suspension having a total concentration of dry e), f) g) and h) in water comprised between 8 and 20% by weight preferably 12% by weight of the total weight of aqueous suspension;
V) The coating B) is applied by nebulizing the aqueous suspension on the coated tablet coming from stage III) previously heated to a temperature comprised between 38 and 45°C, preferably to 42°C, keeping it at the aforementioned temperature until the suspension to be nebulized is exhausted.
